# EUROPÄISCHE PATENTSCHRIFT

(11) **EP 0 880 702 B1**
(45) Veröffentlichungstag und Bekanntmachung des Hinweises auf die Patenterteilung: **03.07.2002**
(21) Anmeldenummer: 96939922.9
(22) Anmeldetag: 26.11.1996
(51) Int. Cl.: G01N 33/68, G01N 33/78, G01N 33/50

(54) **DIAGNOSTISCHES VERFAHREN ZUR BESTIMMUNG DER ÄTIOLOGIE ENTZÜNDLICHER PROZESSE**
DIAGNOSTIC METHOD FOR DETERMINING THE ETIOLOGY OF INFLAMMATORY PROCESSES
PROCEDE DIAGNOSTIQUE POUR DETERMINER L'ETIOLOGIE DE PROCESSUS INFLAMMATOIRES

(30) Priorität: 12.01.1996 DE 19600875
(43) Veröffentlichungstag der Anmeldung: 02.12.1998
(73) Patentinhaber: B.R.A.H.M.S Aktiengesellschaft, 16761 Hennigsdorf (DE)
(72) Erfinder: BOHOUN, Claude, F-75014 Paris (FR)
(74) Vertreter: Andrae, Steffen, Dr.
(86) Internationale Anmeldenummer: EP9605200
(87) Internationale Veröffentlichungsnummer: WO9725622

(56) Entgegenhaltungen:
- DE-C- 4 227 454

## Beschreibung

Die Erfindung betrifft ein diagnostisches Verfahren zum Erkennen der Ursache entzündlicher Prozesse. Entzündliche Prozesse gehören zu den elementaren protektiven Systemleistungen hochorganisierter Organismen und sind eine entscheidende Voraussetzung aller Genesungsprozesse. Die Entzündung ist eine komplexe zelluläre und molekulare Reaktion, mit der die Ausbreitung einer Gewebeschädigung oder eines Infektionserregers eingedämmt und die körperliche Integrität wieder hergestellt werden soll. Die Aktivierung von Monozyten/Makrophagen, die Adhäsion von Granulozyten am Endothel und die chemotaktische Extravasation und Akkumulation von aktivierten Granulozyten, Monozyten und Lymphozyten sind die zentralen Vorgänge in der Pathogenese aller Entzündungsprozesse. Begleiterscheinungen sind die Auslösung von Fieber, die verstärkte Proliferation und Ausschüttung von Zellen der Hämatopoese, die Synthese von Akute-Phase-Proteinen und die Stimulierung der Glucocorticoidsekretion als Teil einer neuroendokrinen Aktivitätssteigerung. Wesentliche Elemente der Reaktion werden durch Zytokine reguliert.

Im Organismus ablaufende Entzündungsprozesse können völlig unterschiedliche Ätiologien haben. Sie können durch eine Blutstrominvasion von Mikroorganismen (Bakterien, Pilze) und/oder ihrer Toxine ausgelöst werden, die zu einer Expression körpereigener proinflammatorischer Substanzen führen. Entzündungsreaktionen können aber auch im Rahmen primär nicht-infektiöser Krankheitsbilder entstehen. Beispiele solcher Erkrankungen sind die hämorrhagisch-nekrotisierende Pankreatitis, das akute Lungenversagen des Erwachsenen (ARDS), Polytraumen mit ausgedehnten tiefen Gewebsnekrosen, Autoimmunkrankheiten und chronische Entzündungen unterschiedlicher Genese.

Die frühzeitige Diagnose der Ätiologie eines Entzündungsprozesses, insbesondere die Unterscheidung, ob es sich um eine durch die Invasion von Mikroorganismen hervorgerufene Entzündung oder ob es sich um nicht-infektiöse Entzündungsprozesse handelt, ist nicht nur für die Prognose des weiteren Krankheitsverlaufes, sondern insbesondere für die Auswahl der therapeutischen Maßnahmen von entscheidender Bedeutung.

Es wurde nun ein diagnostisches Verfahren zur Bestimmung der Ätiologie entzündlicher Prozesse gefunden, bei dem man in einer Probe einer biologischen Flüssigkeit eines Patienten den Gehalt des Peptids Procalcitonin (PCT) und/oder eines daraus gebildeten Teilpeptids, das nicht das reife Calcitonin ist, bestimmt und aus der festgestellten Anwesenheit oder Abwesenheit des bestimmten Peptids auf eine infektiöse oder nicht-infektiöse Ätiologie der Entzündung zurückschließt.

Aus der deutschen Patentschrift 42 27 454 ist bereits ein Verfahren zur Früherkennung, zur Erkennung des Schweregrades sowie zur therapiebegleitenden Verlaufsbeurteilung einer Sepsis bekannt, bei dem man in einer Probe einer biologischen Flüssigkeit eines Patienten den Gehalt des Peptids Procalcitonin und/oder eines daraus gebildeten Teilpeptids, das nicht das reife Calcitonin ist, bestimmt und aus der Anwesenheit des bestimmten Peptids auf das Vorliegen einer Sepsis, ihren Schweregrad und/oder den Erfolg einer therapeutischen Behandlung zurückschließt.

Dort wird auch darauf hingewiesen, daß bei einer normalen viralen Infektion keine oder nur geringfügige Erhöhungen des Procalcitoninspiegels beobachtet werden, so daß durch die Anwendung dieses Diagnostikums eine sichere Unterscheidung zwischen einer Sepsis und einer normalen Viruserkrankung möglich ist.

Es hat sich nun gezeigt, daß eine Erhöhung des Procalcitoninspiegels im Plasma oder Serum auch bei allen nicht-infektiösen Entzündungen unterbleibt, so daß durch Anwendung des Procalcitonintest die unterschiedlichen Ätiologien von äußerlich völlig gleichartig verlaufenden Entzündungsprozessen sicher unterschieden und entsprechend angepaßte Therapien eingeleitet werden können.

Das erfindungsgemäße diagnostische Verfahren hat z.B. große Bedeutung bei der Überwachung des Erfolges von Organtransplantationen.

Tritt nach erfolgter Organtransplantation eine fieberhafte Entzündungsreaktion auf, dann bestand bisher keine Möglichkeit, die wahre Ursache der Entzündung festzustellen. Es kann sich hierbei nämlich entweder um eine durch eingeschleppte Bakterien oder Pilze hervorgerufene Infektion oder aber um eine Abstoßungsreaktion des Organismus gegen das transplantierte Organ handeln. In einer Reihe von klinischen Studien an Patienten mit Herz- oder Lebertransplantationen konnte mit Hilfe des Procalcitonintests eindeutig erkannt werden, ob postoperativ auftretende Entzündungsprozesse durch bakterielle oder durch Pilze verursachte Infektionen hervorgerufen wurden oder ob sie auf Abstoßungsreaktionen zurückzuführen waren. Während im ersteren Fall eine sofortige Antibiotikabehandlung lebensrettend war, mußte bei beginnenden Abstoßungsreaktionen umgehend eine Behandlung mit Immunsuppressiva eingeleitet werden. Da es bei derartigen postoperativen Komplikationen von entscheidender Bedeutung ist, so schnell als möglich mit der richtigen Therapie zu beginnen, zeigt sich in derartigen Fällen der Vorteil des Procalcitonintests besonders eindeutig. Denn die Bestimmung des Procalcitoninspiegels mit Hilfe eines hierfür zur Verfügung stehenden Immunoassays ist einfach, schnell durchführbar und führt zu völlig zuverlässigen Ergebnissen.

Ein anderes Beispiel für die wertvollen diagnostischen Möglichkeiten des Procalcitonintests zeigt sich bei der Untersuchung von Patienten, die an einer akuten Pankreatitis erkranken. Ob eine toxische Pankreatitis vorliegt, die meist auf Alkoholismus zurückzuführen ist, oder ob eine akute, biliäre Pankreatitis zu behandeln ist, bei der durch Gallensteine umfangreiche Gewebsschädigungen und Gewebsentzündungen hervorgerufen werden, konnte bisher nur durch langwierige Untersuchungen entschieden werden. Die Anwendung des Procalcitonintests hingegen ermöglicht eine sehr rasche Entscheidung, weil nur bei der akuten, biliären Pankreatitis der Procalcitoninspiegel im Serum oder Plasma erhöht ist, während die toxische Pankreatitis auf den Procalcitoninspiegel keinerlei Auswirkungen hat.

Auch die Ätiologie schwerster Lungenkomplikationen läßt sich häufig nicht auf einfache Weise bestimmen. ARDS (Adult Respiratory Distress Syndrome) ist eine akute und ernste Änderung in der Lungenstruktur und Lungenfunktion, hervorgerufen durch eine verstärkte Kapillarpermeabilität. Das charakteristische Krankheitssymptom ist starke Atemnot. Hervorgerufen werden kann ARDS sowohl durch zahlreiche Infektionen,vor allem aber durch eine eine starke bakterielle Infektion der Lunge, als auch durch eine Vielzahl von klinischen Zustandsbildern wie Schock, Vergiftung oder Polytrauma. Wird bei einem derartigen Patienten der Procalcitoninspiegel im Plasma oder Serum gemessen, dann zeigen sich bei einer infektiösen Lungenentzündung hohe Procalcitoninwerte, während bei einem durch Schockeinwirkung hervorgerufenen Lungenödem der Procalcitoninwert nicht ansteigt. Die Anwendung des Procalcitonintest ermöglicht somit in einer lebensentscheidenden Phase die schnelle Entscheidung, ob eine Antibiotikabehandlung oder eine Behandlung mit Glukokortikosteroiden angezeigt ist; liegt eine bakterielle Infektion vor, dann würde eine Behandlung mit Glukokortikosteroiden die Infektabwehr unterdrücken und der Patient an der Infektion versterben.

Der kardiogene Schock hat auch heute noch eine extrem hohe Mortalität. Durch neuere Methoden der akuten kardiologischen interventionellen Therapie (PTCA; Stent, IAPB etc.) in Verbindung mit sofortiger kardiochirurgischer Intervention (Bypass, medizinische Assistsysteme) läßt sich die Mortalität verringern. Überraschenderweise versterben jedoch viele dieser zunächst kardial stabilisierten Patienten an Fieber unklaren Ursprungs (FUO) in Verbindung mit reduzierter kardialer Pumpfunktion bzw.erniedrigten peripheren Gefäßwiderständen. Es konnte gezeigt werden, daß PCT (= Procalcitonin) diese Patienten frühzeitig identifiziert, während andere bekannte Inflammationsparameter hierzu nicht in der Lage waren.

Patienten mit Leberzirrhose weisen chronische Verbrauchskoagulopathien auf (Thrombozyten-, AT-III-, Quick- und Fibrinogenerniedrigungen und Nachweis von Fibrinspaltprodukten oder d-Dimeren). Der PCT-Nachweis zum Ausschluß einer bakteriell verursachten Verbrauchskoagulopathie ist in dieser klinischen Situation hilfreich. Verbrauchskoagulopathien treten bei einer Vielzahl von Erkrankungen auf. PCT als sensitiver und spezifischer Marker einer Infektion ist bei den infekt-bedingten Verbrauchskoagulopathien immer deutlich erhöht, jedoch nicht bei Verbrauchskoagulopathien anderer Ursachen.

Die Erregerdiagnostik bei Pneumonien ist sehr häufig negativ. Im Rahmen von PCT-Studien bei Pneumonie-Patienten konnte gezeigt werden, daß bei Pneumokokken-Pneumonie z.T. extrem hohe PCT-Werte im Serum dieser Patienten nachgewiesen wurden. Die Ursache hierfür ist unklar. Möglicherweise stellt das Kapsel-Polysaccharid als wichtigster Virulenzfaktor der Pneumokokken den Stimulus für die PCT-Freisetzung dar. Eine Diagnostik mittels PCT im Serum von Patienten mit Pneumonie stellt eine Bereicherung des bisherigen bekannten diagnostischen Spektrums zur Abklärung der Ätiologie und zur Verlaufskontrolle unter antibiotischer Therapie dar. Es bestehen Hinweise, daß das Kapsel-Polysaccharid der Pneumokokken unter antibiotischer Behandlung vermehrt freigesetzt und der Zustand des Patienten im Sinne einer Jarisch-Herxheimer Reaktion dabei verschlechtert wird. Ein PCT-Anstieg nach Initiierung einer antibiotischen Therapie bei einer Patientin mit Pneumokokkenpneumonie konnte gezeigt werden.

Patienten mit mechanischen Assistsystemen in der Kardiochirurgie zeigen trotz Stabilisierung der Pumpfunktion durch das Assistsystem sehr häufig weiterhin ein katecholaminpflichtiges Schockverhalten. Die Ursache hierfür bleibt in vielen Fällen unklar. Jedoch hat die frühe Detektion einer mikrobiologisch bedingten Kreislaufdepression erhebliche therapeutische und prognostische Konsequenzen. Bekannte Inflammationsparameter, wie CRP, Thrombozyten, Fieber, Leukozyten, IL-6 etc. sind hier nicht in der Lage, eine Infektion anzuzeigen, da diese Parameter infolge des operativen Eingriffs, des Kontaktes mit großen Fremdoberflächen, der Vorbehandlung mit Phosphodiesterasehemmern, sowie immunsuppressiver Behandlung falsch positiv erhöht bzw. erniedrigt sind. Häufig bestehen chronische pulmonale Stauungsinfiltrate, die bei gleichzeitigem Fieber nicht von pneumonisch-entzündlichen Infiltraten zu unterscheiden sind. PCT konnte Patienten mit schweren Infektionen frühzeitig identifizieren, so daß antibiotisches oder chirurgisches Vorgehen geändert wurden. PCT-Positivität und Überleben bis zur Transplantation waren eng korreliert: alle Patienten, die überlebten, hatten ein negatives PCT, während Patienten, die am Multiorganversagen starben, alle hohe PCT-Konzentrationen aufwiesen. Darüber hinaus konnte gezeigt werden, daß das operative Trauma per se nicht zu einer PCT-Freisetzung führte, jedoch einige Patienten postoperativ schwere Infektionen entwickelten.

Ein weiteres wichtiges Indikationsgebiet für die PCT-Bestimmung bei diesen Patienten ist die Erkennung von bakteriellen Infektionen nach längerer Assistabhängigkeit vor einer geplanten Herz-Transplantation. Eine bakterielle oder Pilzinfektion stellt nämlich eine absolute Kontraindikation zur Transplantation dar und konnte mittels erhöhter PCT-Konzentrationen sicher nachgewiesen werden.

Die spontan bakterielle Peritonitis (SDP) ist eine gefürchtete Komplikation bei Patienten mit Aszites bei Leberzirrhose und hat eine hohe Mortalität. Die Diagnose muß früh erfolgen und ist über die mikrobiologische Diagnostik des Aszites häufig erst verspätet möglich (kulturelles Resultat). Diagnostische Kriterien sind außerdem Leukozytenzahlen über 250 - 500/µl und Granulozytenzahlen > 25 - 30% der Aszites-Leukozyten. Allerdings bleibt die Leukozytenzahl allein unsicher, da schon eine diuretische Behandlung zu einem Anstieg der Lymphozytenund damit Gesamtleukozytenzahl im Aszites führen kann. CRP (= C-reaktives Protein) ist als Inflammationsmarker bei Patienten mit Leberzirrhose nur bedingt verwertbar, da CRP hepatisch synthetisiert wird.

Procalcitonin (PCT) wurde in zahlreichen Körperflüssigkeiten untersucht (Liquor, bronchoalveoläre Lavage, Perikarderguß, Pleuraerguß, Magensaft), konnte jedoch in diesen Körperflüssigkeiten in verschiedenen klinischen Situationen und trotz positivem Nachweis im Serum und hoher IL-6 Konzentration (Pleuraerguß, Perikarderguß) nie nachgewiesen werden. Einzig im Aszites war PCT bei Patienten mit spontan bakterieller Peritonitis nachweisbar (oder Peritonitis bei Perforation eines Hohlorgans). Eine Procalcitonin(PCT)-Diagnostik aus Aszites und Serum bei Patienten mit Leberzirrhose kann die Diagnose einer SDP frühzeitig stellen. Bei Leberzirrhose ist der gesamte Dünndarm stark bakteriell besiedelt und eine Translokation durch die ödematöse Dünndarmwand via lymphatisches System in den Aszites möglich.

Procalcitonin (PCT) kann im Ascites von Patienten mit dekompensierter Leberzirrhose nachgewiesen werden, obgleich die Granulozytenzahl pro mm³ noch nicht signifikant erhöht ist. Eine frühzeitige Identifizierung von Risikopatienten ist hiermit möglich.

Bei Patienten mit Peritonitis unterschiedlicher Ätiologien konnte Procalcitonin (PCT) im Ascites nachgewiesen werden und korrelierte mit dem klinischen Verlauf. So konnte eine erhöhte PCT-Konzentration im Ascites bzw. in der Drainageflüssigkeit postoperativer Patienten nach abdominellen operativen Eingriffen nachgewiesen werden.

Bei Autoimmunerkrankungen - besonders unter immunsuppressiven Regimen - ist die Abgrenzung entzündlicher "Schübe" der Grunderkrankung im Status febrilis von bakteriellen Infekten von großer Bedeutung. Die CRP-Bestimmung soll bei dieser Abgrenzung auch dienlich sein. Jedoch sind die Streubreiten groß. So sollen CRP-Werte > 6 mg/L eine bakterielle Infektion anzeigen, Werte < 3 mg/L eine systemische Infektion ausschließen. In der Praxis ist dieser geringe Bereich der Differenzierung jedoch wenig verläßlich. Akute bakterielle Infektionen waren bei diesen Patienten mit einem PCT-Anstieg assoziiert, während dieses bei einer Aktivierung der Grunderkrankung (Schub) nicht der Fall war.

In der Gastroenterologie und Hepatologie kann das CRP als Marker für die Aktivitätskontrolle entzündlicher Darmerkrankungen eingesetzt werden, obwohl es keinen frühzeitigen Hinweis auf einen Rückfall ermöglicht. Bakterielle und Pilzinfektionen sind bei Patienten mit entzündlichen Darmerkrankungen, v.a. unter immunsuppressiver Therapie häufig. PCT-Positivität konnte im Serum von Patienten mit Diarrhoen bakterieller Ursache (z.B. Salmonellen) nachgewiesen werden. Hiermit besteht die Möglichkeit zur Differentialdiagnose der Diarrhoen bei diesen Patienten.

Vor allem in der Pädiatrie ist eine rasche Identifikation des Meningitis-verursachenden Erregers bedeutsam. Wesentlich hierbei ist auch die Unterscheidung von viral vs bakteriell bedingten Meningitiden, da nur bei letzterer Ursache eine antimikrobielle Therapie zur Verfügung steht. Die Verlaufsbeobachtung mittels wiederholter Liquorpunktionen ist gerade bei Kindern aufwendig und stellt eine invasive Maßnahme dar. Es konnte gezeigt werden, daß PCT virale Meningitiden (niedriges PCT) von bakteriell bedingten Formen (hohes PCT) unterscheidet. Auch in der Verlaufsbeobachtung war PCT von klinischer Bedeutung.

Schon bei Verdacht auf eine bakterielle Infektion bei diesen Patienten werden in der klinischen Routine Antibiotika verabreicht. Jedoch ist bei 90% der Kinder, die Antibiotika erhalten, keine Infektion letztlich nachweisbar. CRP identifiziert Patienten "at risk" erst relativ spät, nach > 24 Stunden. Auch können erhöhte CRP-Werte bis 70 mg/L durch das Geburtstrauma, fetale Asphyxie, Schock oder Mekoniumaspiration hervorgerufen weren. Aufwendige erweiterte Labordiagnostik, wie I/T-Quotient des Differentialblutbildes u.a. verlangen weitere Blutabnahmen bei Kindern. PCT ist als schneller Infektionsparameter geeignet, bakterielle Infektionen frühzeitig post partum zu identifizieren.

Für den immunometrischen Assay zum Nachweis von Procalcitonin werden 20 µl Serum oder Plasma benötigt. Nach einer zweistündigen Inkubation bei Raumtemperatur werden die Procalcitonin-Konzentrationen in einem Bereich von 0 bis 500 ng/ml gegen bekannte Standardkonzentrationen ermittelt. Das Serum-Procalcitonin sowie auch die Reagenzien zur Assaydurchführung zeichnen sich durch eine lange Stabilität aus.

Durch die Bestimmung von Procalcitonin in einer biologischen Flüssigkeit eines Patienten läßt sich somit erkennen, ob die vorliegende Entzündung eine infektiöse oder nicht-infektiöse Ursache hat. Darüber hinaus läßt sich durch die Beobachtung der Veränderung des Procalcitoninspiegels im Serum oder Plasma während der Behandlung verfolgen, ob die eingeleiteten Therapiemaßnahmen Erfolg haben oder nicht. Bei einer erfolgreichen Bekämpfung der Infektion wird der Procalcitoninspiegel bald absinken.

Die Bestimmung von Procalcitonin kann grundsätzlich auf verschiedene Weise durchgeführt werden, jedoch hat sich die Bestimmung mittels eines Immuno-Assays, wie er auch in der deutschen Patentschrift 42 27 454 beschrieben ist, besonders bewährt.

Nachfolgend wird die vorliegende Erfindung anhand von praktischen Ergebnissen bei der Behandlung von Patienten mit infektiösen und nicht-infektiösen Entzündungen näher beschrieben.

### Beispiel 1

### Unterscheidung der infektiösen und der nicht-infektiösen Ätiologie des Adult Respiratory Distress Syndroms (=ARDS) mit dem PCT-Nachweis

Bei 17 Patienten mit schweren Lungenverletzungen (ARDS) wurden die inflammatorischen und klinischen Parameter täglich gemessen, einschließlich des PCT-Nachweises mittels eines immunoluminometrischen Assays:

| | Infektiöse ARDS (n=10) | Nicht-infektiöse ARDS (n=7) |
|---|---|---|
| Alter (Jahre) | 58 ± 17 | 44 ± 4 |
| AP-II-Score | 29,66 ± 6,14 | 12,43 ± 2,99 |
| Murray-Score | 2,84 ± 0,23 | 2,76 ± 0,37 |
| PCWm (mmHg) | 10,4 ± 3,3 | 10,32 ± 3,3 |
| Herzleistung (l/min) | 9,2 ± 4 | 9,7 ± 3,4 |
| Neopterin (nmol/L) | 229 ± 225 | 13,5 ± 7,32 |
| IL-6 (pg/ml) | 856,2 ± 889,6 | 704,5 ± 789 |
| TNF-alpha (ng/ml) | 101,7 ± 94,5 | 14,0 ± 15,3 |
| CRP (mg/L | 178,8 ± 122 | 180,8 ± 145,6 |
| PCT (ng/ml) | 36,6 ± 31 | 0,60 ± 0,95 |

Der hohe PCT-Wert bei Patienten mit infektiöser ARDS veranlaßt den Beginn einer Antibiotika-Behandlung,der normale PCT-Wert identifiziert Patienten mit nicht-infektiöser ARDS und veranlaßt zu alternativen therapeutischen Regimen, z.B. zum Einsatz von Glukokortikosteroiden.

### Beispiel 2

Unterscheidung der toxischen von den biliären Ursachen der Pankreatitis

Die frühe Identifizierung einer biliären Ursache hat weitreichende prognostische und therapeutische Konsequenzen. Die bisher verwendeten kliniko-biochemischen Parameter, die zur Diagnostik der biliären Pankreatitis zur Verfügung stehen, haben nur eine geringe Spezifität.

In einer Studie mit 26 Patienten mit akuter Pankreatitis unterschiedlicher Ursachen wurden die klinischen Parameter täglich gemessen, einschließliche eines PCT-Nachweises mittels eines immunoluminometrischen Assays:

| | Biliäre Pankreatitis (n=10) | Toxische Pankreatitis (n=16) |
|---|---|---|
| Alter | 68 ± 13 | 44 ± 16 |
| Geschlecht | 3/7 | 12/4 |
| Anfangs- AP-II Score | 12,5 ± 8 | 5 ± 4,7 |
| Körpertemperatur (°C) | 38,5 ± 0,8 | 38 ± 1,0 |
| Lipase (IU/L) | 1500 ± 2331 | 1394 ± 1488 |
| SGOT (IU/L) | 81 ± 97 | 39 ± 50 |
| SGPT (IU/L) | 135 ± 150 | 48 ± 70 |
| AP (IU/L) | 275 ± 123 | 190 ± 100 |
| Calcium (mmol/L) | 2,17 ± 0,22 | 2,18 ± 0,29 |
| Bilirubin (mg/dl) | 3,8 ± 2,0 | 2,0 ± 1,57 |
| CRP (mg/L) | 253 ± 134 | 346 ± 163 |
| IL-6 (pg/ml) | 913 ± 964 | 905 ± 821 |
| TNF (pg/ml) | 79 ± 97 | 78 ± 109 |
| Neopterin (nmol/L) | 85 ± 110 | 37 ± 53 |
| PCT (ng/ml) | 81 ± 155 | 1,2 ± 1,7 |

Die PCT-Spiegel waren bei Patienten mit biliärer Pankreatitis deutlich erhöht. Dagegen waren die PCT-Werte bei Patienten mit toxischer Pankreatitis niedrig. Hohe PCT-Werte bei Patienten mit akuter Pankreatitis zeigten bakterielle Infektionen an, so daß umgehend eine Therapie mit Antibiotika sowie Maßnahmen zur Entfernung eines Gallengangsteines eingeleitet wurden.

### Beispiel 3

### Identifikation von infektiös bedingten Ursachen des Fiebers

Am Anfang einer Kette von Reaktionen, die den Hypothalamus veranlassen, die Solltemperatur hochzusetzen, stehen exogene Pyrogene verschiedenen Ursprungs. Diese fieberauslösenden Substanzen kommen sowohl bei Infektionen, als auch bei malignen oder immunologisch vermittelten Erkrankungen, als auch bei nicht-infektiös bedingten Entzündungen vor.

Infektiöse Organismen oder deren Abbauprodukte, Antigen-Antikörperkomplexe oder Tumorzellen stimulieren Blutmonozyten oder Gewebsmakrophagen zur Produktion von endogenen Pyrogenen, wie Interleukin 1 und 6, Interferon und TNF. Diese Mediatoren bewirken eine Freisetzung von Prostaglandin E, welches im Hypothalamus die Verstellung der Solltemperatur erreicht.

Fieber ist ein Allgemeinsymptom, dem eine Vielzahl von ätiologischen Faktoren zugrundeliogen mag.

Von kardinaler Bedeutung ist jedoch die Abgrenzung einer infektiven Ursache von anderen, wie Fieber bei malignen Tumoren, Autoimmunerkrankungen, Abstoßungsprozessen bei Transplantatversagen, sogenanntem "Drug fever", "zentralem" Fieber bei primärer Schädigung hypothalamischer Zentren, maligner Hyperthermie nach Anästhesie oder Hitzschlag. In vielen Fällen ist die Identifikation einer zugrundeliegenden Infektion schwierig, darüberhinaus kann sich auf ein nichtinfektiöses Fieber eine Infektion "aufpfropfen". Die Erkennung infektiös bedingten Fiebers hat eine entscheidende therapeutische Konsequenz: die Gabe von Antibiotika. Diese sind zur Behandlung des nichtinfektiös bedingten Fiebers nicht nur nutzlos, sondern unter Umständen sogar schädlich. Akut-Phase-Proteine und Zytokine sind nicht in der Lage, die Ursachen von Fieber zu differenzieren, also nicht hilfreich in dieser Problematik.

### Fall 1:

Ein 30jähriger Mann wird nach einem Kreislaufkollaps bei der Arbeit im Straßenbau im Hochsommer mit Fieber bis 43°C eingeliefert. Die Arbeitsdiagnose ist Hitzschlag. Akut-Phase-Proteine und Zytokine sind massiv erhöht wie bei einer schweren Infektion. Procalcitonin (PCT) ist jedoch lediglich unter 1,0 ng/ml nachweisbar. Der Patient entwickelt jedoch bei weiter bestehendem Fieber von mehr als 40°C am 2. stationären Tag ein beatmungspflichtiges akutes Lungenversagen. PCT ist nunmehr bei 160 ng/ml erhöht nachweisbar. Eine Infektion wird klinisch vermutet, kann jedoch zunächst nicht gesichert werden. Der Patient erhält Antibiotika und überlebt nach dreiwöchiger Intensivtherapie.

### Fall 2:

Eine 48jährige Frau hat seit 3 Wochen Fieber bis 39°C, im Röntgenbild der Thoraxorgane diffuse bilaterale Infiltrate wie bei einer atypischen Pneumonie. C-reaktives Protein, IL-6 und Leukozyten sind deutlich erhöht. Die Patientin wird polychemotherapeutisch antibiotisch behandelt. Der Nachweis einer Infektion kann nicht geführt werden (negative Blutkulturen, Bronchialsekret etc.). Procalcitonin (PCT) ist lediglich im Normalbereich nachweisbar. Die Patientin muß bei Verschlechterung des Allgemeinzustandes maschinell beatmet werden. Trotz massiver respiratorischer Insuffizienz bleibt PCT im täglichen Monitoring im Normalbereich. Eine transbronchiale Lungenbiopsie ergibt schließlich ein Alveolarzellkarzinom, welches nicht behandelbar ist.

### Fall 3:

Ein 45jähriger Mann mit einer Basilaristhrombose wird seit 14 Tagen maschinell beatmet. Er erhält aufgrund einer nosokomialen Pneumonie (Klebsiella pneumoniae) Antibiotika. Alle Entzündungsparameter sind deutlich erhöht, auch Procalcitonin ist mit 18 ng/ml deutlich erhöht. Trotz klinischer Besserung der Pneumonie treten im weiteren Verlauf Temperaturen bis 42°C auf. CRP und IL-6 sind mit einem zweiten peak wieder deutlich erhöht nachweisbar. Procalcitonin ist jedoch mittlerweile wieder in den Normalbereich abgefallen. Auf der Suche nach nichtinfektiösen Ursachen des Fiebers wird ein drug-fever vermutet und Vitamin Bl, das der Patient seit 5 Tagen als neue Medikation intravenös erhält, abgesetzt. Innerhalb 12 Stunden stellt sich wieder eine Normothermie ein.

### Fall 4:

Eine 64jährige Patientin wird nach ambulanter Reanimation aufgrund einer intrazerebralen Blutung und neurochirugischem Notfalleingriff maschinell beatmet. 12 Stunden nach Aufnahme treten Temperaturen bis 43°C auf. "Zentral" induziertes Fieber, also eine nichtinfektiöse Ursache wird vermutet. Das CRP ist zunächst normal, IL-6 ist postoperativ deutlich erhöht. Das Röntgenbild der Thoraxorgane ist unauffällig. Procalcitonin ist jedoch bis auf 30 ng/ml erhöht. Eine Infektion wird nunmehr vermutet. Die Patientin wird bronchoskopiert, es stellt sich eine schwere Aspiration als Ursache des Fiebers heraus. Erst am Folgetag treten im Röntgenbild die zu dieser Diagnose passenden pulmonalen Infiltrate auf, das CRP steigt erst nach 36 Stunden bis auf 40 mg/dl an.

### Beispiel 4

### Unterscheidung von bakteriell/mykotisch/infektiös bedingten vs Tumor-Lyse bedingtem Fieber bei onkologisch-hämatologischen Patienten unter Chemotherapie

Fieber bei onkologisch-hämatologischen Patienten ist häufig schwer interpretierbar und kann Ausdruck des Tumorzerfalls unter der onkologischen Chemotherapie sein. Eine Vielzahl von Zytokinen sind abhängig von Art und Größe des Tumors im Serum dieser Patienten erhöht nachweisbar. Diese Zytokine sind jedoch auch bei bakteriellen Infektionen erhöht. Differentialdiagnostisch hat der Ausschluß von Infektionen jedoch größte Bedeutung, da Patienten unter Chemotherapie erhöht infektanfällig sind.

Bei einer Vielzahl von Patienten konnte der Vorteil von PCT gegenüber anderen Inflammationsparametern in dieser klinischen Situation belegt werden.

### Fall 1

Ein 16jähriger Patient befindet sich ein Jahr nach zytostatischer Behandlung einer akuten myeloischen Leukämie in Vollremission. Die Wiederaufnahme erfolgt wegen Fieber bis 39°C und respiratorischer Insuffizienz. Im Röntgenbild des Thorax diffuse interstitielle Infiltrate. Akut-Phase-Proteine und IL-6 sind deutlich erhöht, im Differentialblutbild Linksverschiebung ohne Leukozytose. Procalcitonin (PCT) ist über den gesamten Verlauf im Normbereich. Ein Erregernachweis gelingt trotz aller Bemühungen (Bronchoskopie, BAL, Blutkulturen etc.) nicht. Unter 3fach-antimikrobieller Therapie und virostatischer Therapie (Acyclovir) verschlechtert sich der Zustand des Patienten. Er wird aufgrund sich verschlechternder Nierenfunktion unter den nephrotoxischen antimikrobiellen Substanzen und zunehmender respiratorischer Partialinsuffizienz auf die Intensivstation verlegt. Bei weiterhin normalen PCT-Werten im Serum werden trotz kritischer klinischer Situation die Antibiotika abgesetzt und der Patient wird nichtinvasiv beatmet (Masken-Bipap). Die Nierenwerte normalisieren sich nach Absetzen der Antibiotika. Nach 4 Tagen unterstützender Beatmung kann der Patient auf die Normalstation zurückverlegt werden. Die pulmonalen Infiltrationen sind spontan rückläufig, sodaß eine Virusinfektion auf dem Boden eines T-Zell-Defektes nach zytostatischer Therapie als Ursache des pulmonalen Versagens angenommen werden muß.

### Fall 2:

Ein 55jähriger Patient mit non-Hodkin-Lymphom entwickelt unter zytostatischer und antimikrobieller Therapie Fieber im Rahmen einer nosokomialen Pneumonie. Bei Aufnahme auf die Intensivstation ist Procalcitonin (PCT) bereits auf 356 ng/ml erhöht. Unter maschineller Beatmung und Katecholaminen verstirbt der Patient 36 Stunden später im refraktären septischen Schock. Eine frühzeitigere PCT-Bestimmung hätte in diesem Fall das Versagen der antimikrobiellen Therapie im damals noch klinisch stabilen Zustand anzeigen können.

### Fall 3:

Ein 14 jähriger Patient mit einer akuten Promyelozytenleukämie enwickelt unter zytostatischer Therapie Fieber, einen Anstieg der Leukozyten unter cis-Retinsäure bis 750.000 TI/nl und bilaterale pulmonale Infiltrate im Röntgen-Thorax. Er wird maschinell beatmungspflichtig und breit antibiotisch behandelt. C-reaktives Protein und IL-6 sind deutlich erhöht, Procalcitonin (PCT) ist jedoch im Normalbereich. Auch im weiteren Verlauf über die nächsten 72 Stunden ist PCT im Serum des Patienten im Normbereich bei fortschreitendem pulmonalen Versagen. Der Patient verstirbt unter den Zeichen einer intrazerebralen Massenblutung im Rahmen eines nicht beherrschbaren cis-Retinoinsäure-Syndroms bei akuter Promyelozytenleukämie. Eine Infektion hat in diesem Fall keine Bedeutung gespielt.

### Beispiel 5

### Erkennung von lebensbedrohlichen bakteriellen oder mykotischen Infektionen bei neutropenischen Patienten unter Chemotherapie

Fieber bei neutropenischen Patienten ist ein häufiges Problem in der Hämatologie und Onkologie. Ursachen können bakterielle, virale oder Pilzinfektionen, aber auch die zytostatische Behandlung, Bluttransfusionen oder die Grunderkrankung als solche sein. Die Diagnostik bakterieller Infektionen ist schwierig, da Leukozytenzahl, Differentialblutbild, Blutsenkungsgeschwindigkeit etc. in dieser Situation wenig aussagekräftig sind und der direkte Nachweis des Erregers sehr schwierig ist. CRP ist bei diesen Patienten mäßig erhöht als Folge des Grundleidens und der Chemotherapie. Angesichts der Dynamik von Infektionen bei diesen Patienten, ist ein "schneller" Parameter essentiell. CRP steigt jedoch erst in den ersten 48-72 Stunden an und ist damit für die sofortige Korrektur (antibiotischer) Therapiemaßnahmen häufig "zu spät". Prophylaktische polyantimikrobielle Therapiemaßnahmen werden daher bei neutropenischen Patienten häufig durchgeführt. Diese haben jedoch bei den durch die Auswirkungen der Chemotherapie bereits kompromittierten Patienten ein hohes Nebenwirkungsspektrum (z.B. Nierenversagen durch Gentamycin, Amphotericin B). PCT steigt nach bakteriellem oder Endotoxinchallenge innerhalb von 3-4 Stunden im Serum an. Daher ist PCT ein geeigneter Marker für onkologische Patienten in der Neutropenie.

### Fall:

Eine 75 jährige Patientin wird 2,5 Jahre nach Remission einer akuten myeloischen Leukämie bei einem Rezidiv erneut stationär aufgenommen und zytostatisch behandelt. Am 18. Tag tritt bei Neutropenie < 100 Fieber bis 39°C unter polyantimikrobieller Therapie und Dyspnoe auf. Im CT des Thorax bronchusnahe Infiltrate, die bei Verdacht auf Aspergilluspneumonie nunmehr zu einer Therapie mit Amphotericin B veranlassen. Trotzdem weitere Verschlechterung des Allgemeinzustandes, sodaß die Patientin intensivtherapeutisch behandelt und maschinell beatmet werden muß. Eine Aspergilluspneumonie kann jedoch nicht bronchoskopisch gesichert werden. Procalcitonin (PCT) ist zunächst nur gering erhöht, ist jedoch wenig später erhöht nachweisbar. Daraufhin werden trotz nur mäßig erhöhten Fiebers Blutkulturen abgenommen, in denen mehrfach koagulase negative Staphylokokken nachgewiesen werden können.

## Patentansprüche

1. Diagnostisches Verfahren zur. Bestimmung der Ätiologie entzündlicher Prozesse, **dadurch gekennzeichnet, daß** man in einer Probe einer biologischen Flüssigkeit eines Patienten den Gehalt des Peptids Procalcitonin (PCT) und/oder eines daraus gebildeten Teilpeptids, das nicht das reife Calcitonin ist, bestimmt und aus der festgestellten Anwesenheit oder Abwesenheit des bestimmten Peptids auf eine infektiöse oder nichtinfektiöse Ätiologie der Entzündung zurückschließt.

2. Verfahren nach Anspruch 1, **dadurch gekennzeichnet, daß** man bei einem Patienten nach erfolgter Organtransplantation, bei dem postoperativ oder im späteren Verlauf entzündliche Erscheinungen auftreten, durch Bestimmung des Procalcitoninspiegeis erkennt, ob die Entzündung durch eine Organabstoßung oder durch einen Infektionsherd verursacht ist.

3. Verfahren nach Anspruch 1, **dadurch gekennzeichnet, daß** man durch die Bestimmung des Procalcitoninspiegels eine akute biliäre Pankreatitis von einer toxischen Pankreatitis unterscheidet.

4. Verfahren nach Anspruch 1, **dadurch gekennzeichnet, daß** man durch die Bestimmung des Procalcitoninspiegels ein durch eine Infektion bedingtes akutes Atemnotsyndrom (ARDS) von einer nicht-infektiös bedingten Schocklunge unterscheidet.

5. Verfahren nach Anspruch 1, **dadurch gekennzeichnet, daß** man durch Bestimmung des PCT-Spiegels bei onkologischen Patienten unterscheidet, ob das auftretende Fieber durch Tumorzellen oder durch eine bakterielle Infektion verursacht wird.

6. Verfahren nach Anspruch 1, **dadurch gekennzeichnet, daß** man durch die Bestimmung des PCT-Spiegels die infektiöse von der nicht-infektiösen Ursache der Verbrauchskoagulopathie von Patienten mit Leberzirrhose unterscheidet.

7. Verfahren nach Anspruch 1, **dadurch gekennzeichnet, daß** man durch die Bestimmung des PCT-Spiegels chronisch-entzündliche Darmerkrankungen von bakteriell-infektiösen Darmerkrankungen unterscheidet.

8. Verfahren nach Anspruch 1, **dadurch gekennzeichnet, daß** man durch die Bestimmung des PCT-Spiegels einen entzündlichen Schub einer Autoimmunerkrankung von einer Infektion unterscheidet.

9. Verfahren nach Anspruch 1, **dadurch gekennzeichnet, daß** man durch die Bestimmung des PCT-Spiegels Medikamenteninduziertes Fieber von einer bakteriell-infektiv bedingten Ursache unterscheidet.

10. Verfahren nach Anspruch 1, **dadurch gekennzeichnet, daß** man durch die Bestimmung des PCT-Spiegels eine bakterielle oder Pilzinfektion als Ursache des hypodynamen Kreislaufverhaltens und der Verbrauchskoagulopathie bei Patienten mit kardialen Unterstützungspumpen frühzeitig erkennt.

11. Verfahren nach Anspruch 1, **dadurch gekennzeichnet, daß** man durch die Bestimmung des PCT-Spiegels im Aszites von Patienten mit Leberzirrhose oder postoperativen Patienten eine Infektion frühzeitig erkennt.

## Claims

1. Diagnostic method for determining the etiology of inflammatory processes, **characterized in that** the content of the peptide procalcitonin (PCT) and/or a partial peptide formed therefrom, which is not mature calcitonin, is determind in a sample of a biological fluid of a patient, and that from the ascertained presence or absence of the determined peptide it is concluded whether the inflammation is of infectious or non-infectious etiology.

2. Method according to claim 1, **characterized in that** it is established by determining the procalcitonin level in a patient who develops, after organ transplantation, inflammatory manifestations postoperatively or in the subsequent course whether the inflammation is caused by organ rejection or by a focus of infection.

3. Method according to claim 1, **characterized in that** the determination of the procalcitonin level is used to distinguish an acute biliary pancreatitis from a toxic pancreatitis.

4. Method according to claim 1, **characterized in that** the determination of the procalcitonin level is used to distinguish an acute respiratory distress syndrome (ARDS) caused by an infection from a shock lung of non-infectious origin.

5. Method according to claim 1, **characterized in that** the determination of the PCT level in oncologic patients is used to distinguish whether the emergent fever is caused by tumor cells or by a bacterial infection.

6. Method according to claim 1, **characterized in that** the determination of the PCT level is used to distinguish, in a patient with cirrhosis of the liver, a disseminated intravascular coagulation of infectious origin from one of non-infectious origin.

7. Method according to claim 1, **characterized in that** the determination of the PCT level is used to distinguish chronic inflammatory intestinal diseases from bacterial-infectious intestinal diseases.

8. Method according to claim 1, **characterized in that** the determination of the PCT level is used to distinguish an inflammatory flare-up of an autoimmune disease from an infection.

9. Method according to claim 1, **characterized in that** the determination of the PCT level is used to distinguish a drug-induced fever from a fever of bacterial-infectious origin.

10. Method according to claim 1, **characterized in that** the determination of the PCT level is used to early recognize a bacterial or mycotic infection as the cause of hypodynamic circulatory behaviour and disseminated intravascular coagulation in patients with cardiac support pumps.

11. Method according to claim 1, **characterized in that** the determination of the PCT level in the ascites of patients with cirrhosis of the liver, or in postoperative patients, is used to early recognize an infection.

## Revendications

1. Procédé de diagnostic pour la détermination de l'étiologie de processus inflammatoires, **caractérisé en ce que** l'on détermine la teneur du peptide procalcitonine (PCT) et/ou d'un peptide partiel formé à partir de celui-ci, qui n'est pas la calcitonine mature, dans un échantillon d'un fluide biologique d'un patient, et **en ce que**, à partir de la présence ou de l'absence détectée du peptide déterminé, on détermine si l'étiologie de l'inflammation est infectieuse ou non infectieuse.

2. Procédé selon la revendication 1, **caractérisé en ce que** l'on établit, par détermination du taux de procalcitonine chez un patient qui développe, après une transplantation d'organe, des phénomènes inflammatoires après l'opération ou ultérieurement, si l'inflammation est provoquée par un rejet de l'organe ou par un foyer d'infection.

3. Procédé selon la revendication 1, **caractérisé en ce que** la détermination du taux de procalcitonine est utilisée pour distinguer une pancréatite biliaire aiguë d'une pancréatite toxique.

4. Procédé selon la revendication 1, **caractérisé en ce que** la détermination du taux de procalcitonine est utilisée pour distinguer un syndrome de détresse respiratoire aiguë (ARDS: acute respiratory distress syndrome) provoqué par une infection, d'un choc pulmonaire d'origine non infectieuse.

5. Procédé selon la revendication 1, **caractérisé en ce que** la détermination du taux de PCT chez des patients cancéreux est utilisée pour distinguer si une l'apparition de fièvre est provoquée par des cellules tumorales ou par une infection bactérienne.

6. Procédé selon la revendication 1, **caractérisé en ce que** la détermination du taux de PCT est utilisée pour distinguer, chez des patients atteints de cirrhose du foie, l'origine infectieuse ou non infectieuse d'une coagulation intravasculaire disséminée.

7. Procédé selon la revendication 1, **caractérisé en ce que** la détermination du taux de PCT est utilisée pour distinguer des maladies inflammatoires chroniques de l'intestin de maladies infectieuses bactériennes de l'intestin.

8. Procédé selon la revendication 1, **caractérisé en ce que** la détermination du taux de PCT est utilisée pour distinguer un choc inflammatoire d'une maladie autoimmune d'une infection.

9. Procédé selon la revendication 1, **caractérisé en ce que** la détermination du taux de PCT est utilisée pour distinguer une fièvre induite par médicaments d'une fièvre due à une infection bactérienne.

10. Procédé selon la revendication 1, **caractérisé en ce que** la détermination du taux de PCT est utilisée pour reconnaître précocement, chez des patients munis de pompes de soutien cardiaque, une infection par des bactéries ou des champignons comme cause d'une circulation hypodynamique et d'une coagulation intravasculaire disséminée.

11. Procédé selon la revendication 1, **caractérisé en ce que** la détermination du taux de PCT dans les ascites de patients atteints de cirrhose du foie ou de patients venant de subir une opération, est utilisée pour reconnaître précocement une infection
